# EUROPEAN PATENT SPECIFICATION

(11) **EP 2 948 076 B1**
(45) Date of publication and mention of the grant of the patent: **31.05.2017**
(21) Application number: 14705241.9
(22) Date of filing: 27.01.2014
(51) Int. Cl.: A61B 17/29, A61B 18/14, A61M 25/01, A61B 17/3205, A61B 18/00, A61B 17/00

(54) **A COUPLING MECHANISM FOR A MEDICAL DEVICE**
KOPPLUNGSMECHANISMUS FÜR EINE MEDIZINISCHE VORRICHTUNG
MÉCANISME D'ACCOUPLEMENT DE DISPOSITIF MÉDICAL

(30) Priority: 28.01.2013 US 201361757508 P
(43) Date of publication of application: 02.12.2015
(73) Proprietor: Boston Scientific Scimed, Inc., Maple Grove, MN 55311 (US)
(72) Inventor: SMITH, Paul, Smithfield, RI 02917 (US); ISAACSON, Brad, M., Lancaster, MA 01523 (US); TAT, Eric, Canton, MA 02021 (US); SUON, Naroun, Lawrence, MA 01843 (US); RAYBIN, Samuel, Marlborough, MA 01752 (US)
(74) Representative: Vossius & Partner Patentanwälte Rechtsanwälte mbB
(86) International application number: PCT/US2014/013179
(87) International publication number: WO 2014/117078

(56) References cited:
- EP-A2- 2 133 028
- US-A- 5 582 615
- US-A1- 2009 171 147
- US-A1- 2012 197 190

## Description

### Field of the Disclosure

Embodiments of the present disclosure include medical devices, and more particularly, medical devices including an elongate member and a coupling mechanism configured to connect and disconnect portions of the elongate member.

### Background of the Disclosure

Organ walls are composed of several layers: the mucosa (the surface layer), the submucosa, the muscularis (muscle layer), and the serosa (connective tissue layer). In gastrointestinal, colonic, and esophageal cancer, e.g., small polyps or cancerous masses may form along the mucosa and often extend into the lumens of the organs. Conventionally, this condition is treated by cutting out a portion of the affected organ wall. This procedure, however, may cause discomfort to patients, and pose health risks. Recently, physicians have adopted a minimally invasive technique called endoscopic mucosal resection (EMR), and another called endoscopic submucosal dissection (ESD), which removes the cancerous or abnormal tissues (e.g., polyps), keeping the walls intact. EMR may also assist in removing any undesired tissue, even if such tissue is not abnormal or diseased.

EMR and ESD are generally performed with an endoscope, which may be a long, narrow elongate member optionally equipped with a light, imaging device, and other instruments and defining one or more lumens extending from a proximal to a distal end of the elongate member. During ESD, the endoscope is passed down the throat or guided through the rectum to reach an undesired tissue, such as a polyp, in an affected organ. The distal end of the endoscope, typically equipped with a hood carrying dissecting tools is guided towards the undesired tissue. The hood is also typically used to create a working volume, applying tissue tension for endoscopic instruments, and preventing extraneous tissue and debris from interfering with the operator's visualization and operation. For EMR, the undesired tissue may be drawn into the hood. This may be achieved by applying suction through working channel extending along the lumen, or by retracting a retraction tool that is extendable from the endoscope. When the undesired tissue is sufficiently drawn into the hood, the dissecting tool may dissect portions of the tissue or resect target tissue from the organ wall. Subsequently, the excised tissue may be extracted for examination, biopsy, or disposal.

In general, the dissection tools include a distal tool, such as a cap, a hood, a wire loop, a band, or a knife, configured to dissect or resect target tissue, a proximal handle configured to control actuation of the distal tool, and an elongate member operably connecting the distal tool and the proximal handle. The dissection tools are delivered through a lumen of the endoscope to the target tissue. In some applications, however, certain distal tools and/or proximal handles may be too large to be introduced through an endoscope lumen. Accordingly, there exists a need for an improved dissection tool for accommodating endoscope lumens of various sizes.

### Summary of the Disclosure

In accordance with an embodiment, a medical device may include a proximal handle, a distal tool, an elongate member connecting the distal tool to the proximal handle and including a first portion and a second portion, wherein the elongate member may be configured to deliver electrical energy to the distal tool, and a coupling mechanism configured to connect and disconnect the first and second portions of the elongate member.

Various embodiments of the disclosure may include one or more of the following aspects: wherein the first portion of the elongate member may be connected to the distal tool, and the second portion of the elongate member may be connected to the proximal handle, wherein the coupling mechanism may include an electrically-conductive material and may be configured to deliver electrical energy between the first and second portions of the elongate member, wherein the coupling mechanism may include a linkage apparatus coupled to the first portion of the elongate member and a holding apparatus coupled to the second portion of the elongate member, and wherein the linkage apparatus may be configured to engage and disengage with the holding apparatus, wherein the holding apparatus may define a lumen into which the linkage apparatus is configured to be inserted, wherein the holding apparatus may be configured to restrict movement of the linkage apparatus within the lumen of the holding apparatus, wherein the holding apparatus may include a movable plate configured to hold a position of the linkage apparatus within the lumen of the holding apparatus, wherein the holding apparatus may include a first slot and a second slot each defined on a side surface of the holding apparatus and opening into the lumen, and wherein the linkage apparatus may include a stem and a boss having a size larger than a size of the stem, wherein the boss and the stem may be configured to be delivered into the lumen of the holding apparatus through the first and second slots, and wherein the boss may be restricted from movement by the walls of the holding apparatus defining the first and second slots, wherein the linkage apparatus may include a protrusion configured to form a friction fit with an inner wall of the holding apparatus defining the lumen, and a jacket configured to be advanced over the coupling mechanism, wherein the jacket may comprise an insulating material.

In accordance with another embodiment, a medical device may include a proximal handle, a distal tool, an elongate member including a first portion connected to the distal tool and a second portion connected to the proximal handle, and a coupling mechanism including a linkage apparatus and a holding apparatus and configured to connect and disconnect the first and second portions of the elongate member, wherein the holding apparatus may include at least one slot defined on a side surface of the holding apparatus and opening into a lumen of the holding apparatus, and wherein the linkage apparatus may be configured to be inserted through the at least one slot of the holding apparatus and secured within the lumen.

Various embodiments of the disclosure may include one or more of the following aspects: wherein the coupling mechanism may include an electrically-conductive material, wherein the coupling mechanism may be configured to provide an electrical pathway from the proximal handle to the distal tool when the first and second portions of the elongate member are connected together, and wherein the at least one slot may include a first slot and a second slot, the linkage apparatus may include a stem and a boss having a size larger than a size of the stem, and wherein the stem and the boss may be configured to be inserted through the first slot and the second slot.

In accordance with yet another embodiment, a medical device may include a proximal handle, a distal tool, an elongate member connecting the distal tool to the proximal handle and including a first portion and a second portion, and a coupling mechanism including a holding apparatus and a linkage apparatus and configured to connect and disconnect the first and second portions of the elongate member, wherein the holding apparatus may define a lumen into which the linkage apparatus may be configured to be inserted, and wherein the holding apparatus may include a movable plate configured to hold a position of the linkage apparatus within the lumen of the holding apparatus.

Various embodiments of the disclosure may include one or more of the following aspects: wherein the linkage apparatus may include an electrically-conductive material and may be configured to deliver electrical energy between the first and second portions of the elongate member, wherein the holding apparatus may include an insulating material incapable of conducting electricity, wherein the linkage apparatus may be connected to the first portion of the elongate member, and the holding apparatus may be connected to the second portion of the elongate member, and wherein the linkage apparatus may abut the second portion of the elongate member within the lumen of the holding apparatus, and wherein the movable plate may intersect the lumen of holding apparatus and may include a slot configured to abut against the linkage apparatus.

### Brief Description of the Drawings

Fig. 1 is a schematic illustration of an endoscopic device, according to an exemplary disclosed embodiment;
Fig. 2 is a perspective view of a medical device for use with the endoscopic device of Fig. 1, according to an exemplary disclosed embodiment;
Fig. 3A illustrates a perspective view of a coupling mechanism for the medical device of Fig. 2, according to an exemplary disclosed embodiment;
Fig. 3B illustrates another perspective view of the coupling mechanism of Fig. 3A, according to an exemplary disclosed embodiment;
Fig. 3C illustrates yet another perspective view of the coupling mechanism of Fig. 3A, according to an exemplary disclosed embodiment;
Fig. 4A illustrates a perspective view of another coupling mechanism for the medical device of Fig. 2, according to an exemplary disclosed embodiment;
Fig. 4B illustrates a front view of a locking component of the coupling mechanism of Fig. 4A, according to an exemplary disclosed embodiment;
Fig. 4C illustrates a side view of the locking component of the coupling mechanism of Fig. 4A, according to an exemplary disclosed embodiment;
Fig. 5 illustrates a perspective view of another coupling mechanism for the medical device of Fig. 2, according to an exemplary disclosed embodiment;
Fig. 6 illustrates a perspective view of another coupling mechanism for the medical device of Fig. 2, according to an exemplary disclosed embodiment; and
Figs. 7A and 7B schematically illustrate the proximal delivery of an elongate member of the medical device of Fig. 2 through an endoscopic device, according to an exemplary disclosed embodiment.

### Detailed Description

Reference will now be made in detail to exemplary embodiments of the present disclosure described above and illustrated in the accompanying drawings. Wherever possible, the same reference numbers will be used throughout the drawings to refer to the same or like parts.

The terms "proximal" and "distal" are used herein to refer to the relative positions of the components of an endoscopic device 100 and a medical device 1. When used herein, "proximal" refers to a position relatively closer to the exterior of the body or closer to an operator, such as a surgeon, using endoscopic device 100 or medical device 1. In contrast, "distal" refers to a position relatively further away from the operator using endoscopic device 100 or medical device 1, or closer to the interior of the body.

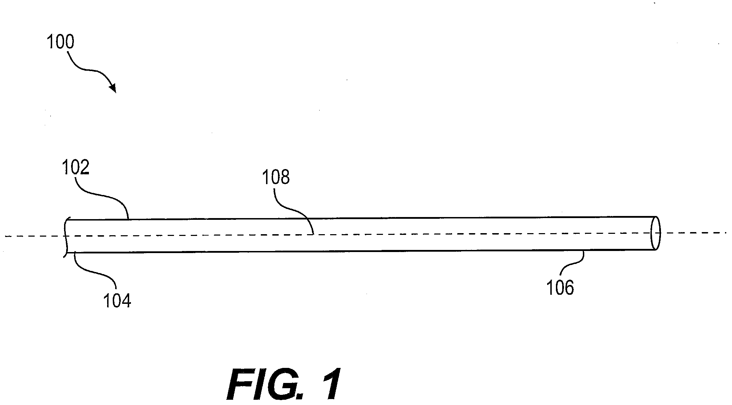

Fig. 1 illustrates a schematic view of an exemplary endoscopic device 100 of the present disclosure. Endoscopic device 100 may be used for dissecting and/or resecting polyps, lesions, or other unwanted tissue from the interior bodily walls of a patient, or for any other medical procedure. Endoscopic device 100 may include an elongate member 102 having a proximal end 104, a distal end 106, and a lumen 108 extending between proximal and distal ends 104, 106. Proximal end 104 may be coupled to an appropriate handle (not shown), while distal end 106 may include one or more openings in communication with lumen 108.

Endoscopic device 100 may be an endoscope or any other suitable introduction device or sheath adapted to be advanced into a body lumen. For example, endoscopic device 100 may include a guide tube, an endoscope, a guide sheath, a flexible member, and/or a catheter. In the illustrated embodiment, lumen 108 of elongate member 102 may include one or more channels (not shown), through which, the operator may introduce one or more medical devices to extend out of distal end 106 of elongate member 102. For example, during a resection, the operator may introduce a suction device into one channel and a cutting device, such as, for example, a snare loop, into another channel. Additionally, from time to time during the procedure, the operator may insert a light source, a camera, an injector, or a morcellator within the one or more channels. Because different implements may need to be inserted into the elongate member 102, the dimensions of its channels may vary. Some channels may have a larger diameter, while others may have a smaller diameter. Further, some channels may include permanently fixed medical devices, such as light sources or imaging devices, while other channels may allow temporary insertion and removal of medical devices, as the operator desires. In addition or alternatively, a camera and/or light sources may be integral with the elongate member. Elongate member 102 may also be any known endoscopic device used, for example, for colonoscopy, resectoscopy, cholangioscopy, or mucosal resection.

Moreover, elongate member 102 may be coated with lubricious materials and antibacterial agents to ease insertion into tight cavities and prevent infections, respectively. In addition, elongate member 102 may comprise surface designs. Further, portions of the elongate member 102 may include radiopaque materials to visualize the position of elongate member 102 within a patient's body.

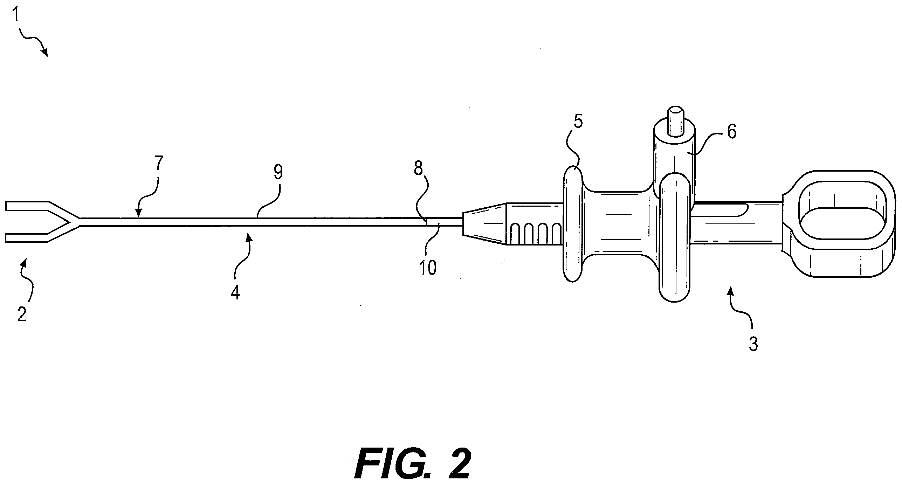

Fig. 2 illustrates a perspective view of medical device 1 for use with endoscopic device 100, according to an exemplary embodiment. Medical device 1 may include a distal tool 2, a proximal handle 3, and an elongate member 4 operably connecting distal tool 2 to proximal handle 3. In the exemplary embodiments of this disclosure, medical device 1 may include any suitable device for resection and/or dissection procedures. For example, distal tool 2 may comprise a cap configured to guide suction and cutting of tissue for an EMR procedure, and may include one or more features of the caps disclosed in U.S. Provisional Application No. 61/581,735, which is incorporated herein by reference in its entirety. In other embodiments, distal tool 2 may comprise a hood configured to accommodate one or more medical devices and having one or more articulating sections configured to create a working volume for an ESD procedure. In such embodiments, the hood may include one or more features of the hoods disclosed in U.S. Provisional Application No. 61/602,372. Moreover, distal tool 2 may comprise any other tool for resection and/or dissection procedures, such as, for example, a wire loop, a snare loop, a band, or a knife. In addition, distal tool 2 may comprise any suitable electrically-conductive material. It should be appreciated, however, that medical device 1 is not limited to resection and dissection devices, and may include any other suitable instrument or tool for any medical procedure.

Elongate member 4 may couple distal tool 2 to proximal handle 3 and may effectuate axial movement of distal tool 2, or a portion thereof (such as a snare loop within distal tool 2), relative to a longitudinal axis of elongate member 4. For example, an actuating wire within elongate member 4 may be coupled to a slidable spool 5 of proximal handle 3, wherein distal advancement of spool 5 may distally advance distal tool 2 relative to a fixed portion of proximal handle 3, and proximal retraction of spool 5 may proximally retract distal tool 2 relative to the fixed portion of proximal handle 3. It should be appreciated that any other suitable handle arrangement may be employed to actuate movement of tool 2, or a portion of tool 2, relative to elongate member 4, in any direction.

Moreover, an actuating wire within elongate member 4 may comprise an electrically-conductive material and may be coupled to an electrical port 6 of proximal handle 3. Electrical port 6 may in turn be coupled to a suitable electrical source (not shown) and may be configured to deliver electrical energy from the electrical source to the actuating wire. The electrical energy may then travel through elongate member 4 and to distal tool 2, or a portion of tool 2, such as a snare loop.

Medical device 1 may also include a suitable insulating material 7 coated along the entirety of elongate member 4. Insulating material 7 may be incapable of conducting energy to shield and prevent the discharge of stray electrical energy from elongate member 4. Additionally, or alternatively, elongate member 4 may be formed of insulating material 7.

In certain other embodiments, elongate member 4 may be directly coupled to slidable spool 5 of proximal handle 3, wherein distal advancement of spool 5 may distally advance elongate member 4 and distal tool 2 relative to a fixed portion of proximal handle 3, and proximal retraction of spool 5 may proximally retract elongate member 4 and distal tool 2 relative to the fixed portion of proximal handle 3. Furthermore, elongate member 4 may comprise an electrically-conductive material and may be coupled to electrical port 6 of proximal handle 3. Electrical port 6 may in turn be coupled to a suitable electrical source (not shown) and may be configured to deliver electrical energy from the electrical source to elongate member 4. The electrical energy may then travel through elongate member 4 and to distal tool 2. In such embodiments, insulating material 7 may also be coated along the entirety of elongate member 4.

Medical device 1 may also include a coupling mechanism 8 configured to removably attach distal tool 2 to proximal handle 3. More particularly, elongate member 4 may include a first portion 9 and a second portion 10 configured to operably connect together and disconnect from each other at coupling mechanism 8. Distal tool 2 may be coupled to first portion 9, and proximal handle 3 may be coupled to second portion 10. As illustrated in the exemplary embodiment of Fig. 2, coupling mechanism 8 may be disposed on elongate member 4 proximate proximal handle 3. Accordingly, first portion 9 may include a length larger than a length of second portion 10. When medical device 1 is in a disassembled configuration (i.e., first portion 9 and second portion 10 of elongate member 4 are disconnected), first portion 9 may be delivered through a working channel of elongate member 102 via an opening at distal end 106. First portion 9 may then exit the working channel through an appropriate opening at proximal end 104 of elongate member 102, such as a port on elongate member 102, or may be delivered through a port or opening on the handle assembly (not shown) of endoscopic device 100. Once delivered out of the working channel, first portion 9 may be connected to second portion 10 by coupling mechanism 8. Consequently, proximal handle 3 may be operably coupled to distal tool 2, with proximal handle 3 external the working channel and disposed near proximal end 104 of elongate member 102, and distal tool 2 external the working channel and disposed near distal end 106 of elongate member 102.

In certain other embodiments, coupling mechanism 8 may be disposed on elongate member 4 proximate distal tool 2. As such, first portion 9 connected to distal tool 2 may include a length smaller than a length of second portion 10 connected to proximal handle 3. In such embodiments, second portion 10 may be delivered through a working channel of elongate member 102 via an appropriate opening at proximal end 104 of elongate member 102, or a port or opening on the handle assembly (not shown) of endoscopic device 100. Second portion 10 may exit the working channel through an opening at distal end 106 of elongate member 102, and may then be connected to first portion 9 by coupling mechanism 8. Similar to the embodiment discussed above, proximal handle 3 may therefore be operably coupled to distal tool 2, with proximal handle 3 external the working channel and disposed near proximal end 104 of elongate member 102, and distal tool 2 external the working channel and disposed near distal end 106 of elongate member 102.

Additionally, or alternatively, medical device 1 may include more than one coupling mechanism 8. For example, a first coupling mechanism 8 may be disposed on elongate member 4 proximate proximal handle 3, and a second coupling mechanism 8 may be disposed proximate distal tool 2. Accordingly, portions of elongate member 4 may be selectively connected and disconnected to facilitate the delivery of medical device 1 through elongate member 102. In other words, when elongate member 4 is disassembled at the first coupling mechanism 8, a portion of elongate member 4 coupled to distal tool 2 may be delivered through a working channel of elongate member 102 via an opening at distal end 106, and elongate member 4 may be reassembled near proximal end 104 of elongate member 102. Or, if desired, elongate member 4 may be disassembled at the second coupling mechanism 8, a portion of elongate member 4 coupled to proximal handle 3 may then be delivered through a working channel of elongate member 102 via an opening near proximal end 104, and elongate member 4 may be reassembled at distal end 106 of elongate member 102.

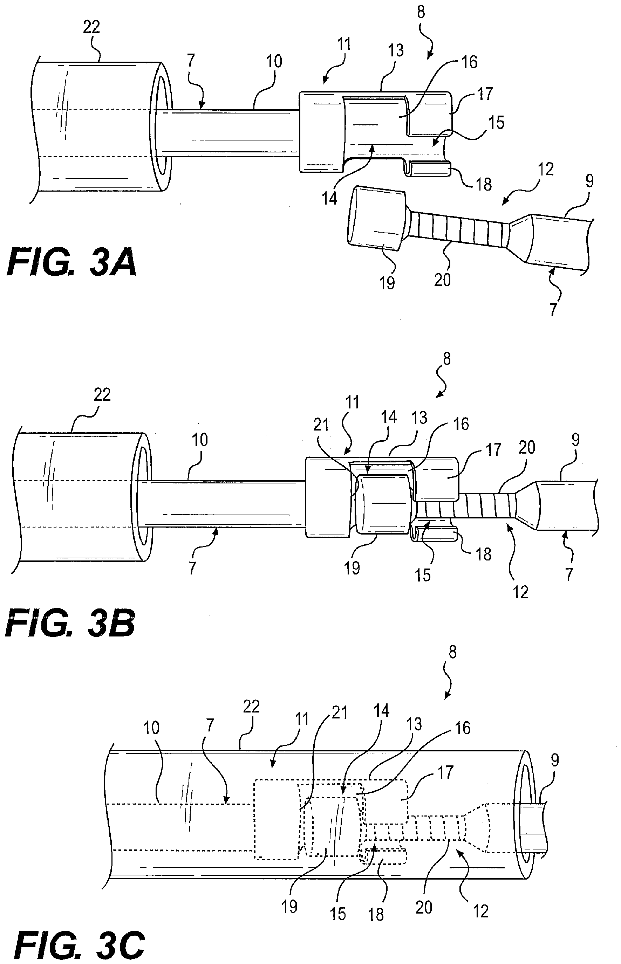

Fig. 3A illustrates a perspective view of coupling mechanism 8, according to an exemplary disclosed embodiment. Coupling mechanism 8 may include a holding apparatus 11 connected to second portion 10 of elongate member 4 and a linkage apparatus 12 connected to first portion 9 of elongate member 4. Holding apparatus 11 may be configured to engage and disengage with linkage apparatus 12, and thus, operably couple and disconnect proximal handle 3 and distal tool 2.

Holding apparatus 11 may comprise a generally cylindrically-shaped member 13 including a first slot 14 and a second slot 15 each disposed on a side surface of cylindrically-shaped member 13. First slot 14 may be larger than second slot 15 and may open into a lumen 16 of cylindrically-shaped member 13. Second slot 15 may also open into lumen 16 of cylindrically-shaped member 13, and may be defined by a first arm 17 and a second arm 18 of cylindrically-shaped member 13. Second slot 15 may open on a proximal side into first slot 14 and may open on a distal side into a distal face of cylindrically-shaped member 13.

Holding apparatus 11 may be connected to second portion 10 of elongate member 4 by any suitable mechanism, such as, for example, a weld, adhesives, fasteners, and the like. Alternatively, holding apparatus 11 and second portion 10 may be integrally formed as a single piece. In addition, holding apparatus 11 may comprise any suitable electrically-conductive material to transport electrical energy from the electrical source (not shown) and second portion 10 to linkage apparatus 12. In certain embodiments, holding apparatus 11 and second portion 10 may be a single, continuous piece of the electrically-conductive material.

Linkage apparatus 12 may include a boss 19 connected to a stem 20. Boss 19 may include an appropriate size and shape to fit through first slot 14 (and not through second slot 15) and be positioned within lumen 16 of holding mechanism 11. For example, boss 19 may have a substantially cylindrical shape having a diameter smaller than a width of first slot 14 measured around a circumference of cylindrically-shaped member 13. Boss 19 may be larger than lumen 16 and smaller than slot 14, such that boss 19 may move within lumen 16 due to slot 14 and may not move within lumen 16 beyond the end of slot 14. It is contemplated that boss 19 may have any suitable shape, e.g., cubical, rectangular, pyramidal, oval, spherical, conical, irregular, uniform, and may be any suitable size configured to fit within first slot 14. It is also contemplated that first slot 14 may include a complimentary shape to that of boss 19. For example, if boss 19 is conical, first slot 14 may have a complimentary conical shape such that boss 19 matingly engages first slot 14. Other shapes are also contemplated, e.g., a ball and socket connection, a snap-fit connection, and/or other suitable shapes. All such shapes may increase the contact between holding mechanism 11 and linking apparatus 12 and may increase electrical contact between them.

Similarly, stem 20 may include an appropriate size and shape to fit through second slot 15 and be positioned within and configured to move relative to lumen 16 of holding mechanism 11. Accordingly, boss 19 may have a diameter larger than a diameter of stem 20, and boss 19 may be restricted from entering second slot 15 since the diameter of boss 19 may be larger than a width of second slot 15. It is contemplated that second slot 15 may be radially offset from first slot 14. As such boss 19 may fit within slot 14 and linking apparatus 12 may then be rotated relative to holding mechanism 11 to fit neck 20 within slot 15. Such an arrangement may avoid linking apparatus 12 from inadvertently disconnecting from holding mechanism 11 because first and second slots 14, 15 are not radially aligned with boss 19 and neck 20.

Similar to holding apparatus 11, linkage apparatus 12 may be connected to first portion 9 of elongate member 4 by any suitable mechanism, such as, for example, a weld, adhesives, fasteners, and the like. Moreover, linkage apparatus 12 may comprise any suitable electrically-conductive material to transport electrical energy from holding apparatus 11 to first portion 9 and distal tool 2. In certain embodiments, linkage apparatus 12 and first portion 9 may be a single, continuous piece of the electrically-conductive material.

Fig. 3B illustrates another perspective view of coupling mechanism 8 with linkage apparatus 12 engaged to holding apparatus 11, according to an exemplary embodiment. As alluded to above, when coupling linkage apparatus 12 to holding apparatus 11, boss 19 may be delivered through first slot 14 and positioned within lumen 16 of holding apparatus 11, and stem 20 may be delivered through second slot 15 and positioned within lumen 16 of holding apparatus 11. In addition, one or both of boss 19 and stem 20 may be in contact with holding apparatus 11 when positioned within lumen 16 to provide an electrical connection between holding apparatus 11 and linkage apparatus 12.

As shown in Fig. 3B, boss 19 may be restricted from axial movement relative to lumen 16 by the walls of holding apparatus 11 defining first and second slots 14, 15. First and second arms 17, 18 may have suitable thicknesses (measured radially from an outer surface of cylindrically-shaped member 13 to lumen 16) to prevent boss 19 from axially exiting lumen 16 in a distal direction, and a proximal wall 21 of holding apparatus 11 may block axial translation of boss 19 in a proximal direction. Accordingly, as second portion 10 of elongate member 4 is proximally retracted by actuation of proximal handle 3, cylindrically-shaped member 13 may axially move relative to stem 20, and first and second arms 17, 18 may abut against a distal end of boss 19. Further proximal retraction of second portion 10 may consequently cause first and second arms 17, 18 to exert a proximal force on boss 19 and thereby retract first portion 9 of elongate member 4 and distal tool 2 in the proximal direction. Moreover, as second portion 10 of elongate member 4 is distally advanced by actuation of proximal handle 3, proximal wall 21 may abut against a proximal end of boss 19, and consequently, advance first portion 9 of elongate member 4 and distal tool 2 in the distal direction.

With reference to Figs. 3A and 3B, coupling mechanism 8 may also include a jacket 22. Jacket 22 may include a tubular member comprising a suitable electrically-insulating material incapable of conducting electricity. Jacket 22 may be configured to be advanced over coupling mechanism 8 and may be retracted to expose coupling mechanism 8.

Fig. 3C illustrates yet another perspective view of coupling mechanism 8 with jacket 22 positioned over coupling mechanism 8, according to an exemplary embodiment. When positioned over coupling mechanism 8, jacket 22 may insulate coupling mechanism 8 to shield and prevent the discharge of stray electrical energy. In addition, a lumen of jacket 22 may include an appropriate diameter to allow axial movement of coupling mechanism 8 and elongate member 4, but block radial displacement of one or both of boss 19 and stem 20 respectively through first and second slots 14, 15. First portion 9 of elongate member 4, second portion 10 of elongate member 4, holding apparatus 11, and linkage apparatus 12 may axially move relative to jacket 22, with undesired disconnection between holding apparatus 11 and linkage apparatus 12 prevented by jacket 22. It should be appreciated that jacket 22 may include a suitable locking mechanism, such as, for example, removable straps, removable fasteners, and the like, to change and hold an axial position of jacket 22 relative to coupling mechanism 8. The locking mechanism may be coupled to proximal handle 3 to allow relative movement between elongate member 4 and jacket 22.

In some embodiments, selective portions of holding apparatus 11 and linkage apparatus 12 may be coated with an insulating material. For example, an entirety of holding apparatus 11 and linkage apparatus 12 may be coated with the insulating material except for certain contacting surfaces between holding apparatus 11 and linkage apparatus 12. Those contacting surfaces may provide the electrical connection between first portion 9 and second portion 10 of elongate member 4. One contacting surface may be defined on an interior surface of lumen 16 of holding apparatus 11, and another contacting surface may be defined on a surface of boss 19 of linkage apparatus 12 that may contact the contacting surface in lumen 16. Accordingly, an exterior surface of coupling mechanism 8 may be insulated and may prevent undesired electrical discharges to the external environment. In addition, holding apparatus 11 and linkage apparatus 12 may be connected together via a friction or interference fit, such that linkage apparatus 12 may be prevented from readily disengaging with holding apparatus 11 through, for example, first and second slots 14, 15. In these embodiments, jacket 22 may or may not be employed. In addition, an insulative coating on and/or tubular element within coupling mechanism 8 may help is locking the holding apparatus 11 and linking apparatus 12 together by forming an interference or snap-fit type of joint.

In certain other embodiments, coupling mechanism 8 may be associated with an actuating wire (not shown) within elongate member 4, and may be configured to connect and disconnect portions of the actuating wire. The actuating wire may include a first section connected to distal tool 2, or a portion of distal tool 2, and extending through a lumen defined by first portion 9 and a second section connected to proximal handle 3 and extending through a lumen defined by second portion 10. Holding apparatus 11 may be connected to the second section of the actuating wire, and linkage apparatus 12 may be connected to the first section of the actuating wire. Similar to the embodiments discussed above, linkage apparatus 12 may be configured to engage and disengage with holding apparatus 11 to operably couple the actuating wire. For example, actuation of proximal handle 3 may move the actuating wire and distal tool 2 relative to elongate member 4, and an electrical connection may be formed between the electrical source (not shown), the actuating wire, coupling mechanism 8, and distal tool 2. Moreover, in such embodiments, jacket 22 may be slid over coupling mechanism 8, and may form a friction fit between first portion 9 and second portion 10 of elongate member 4. The lumen of jacket 22 may, however, include an appropriate diameter to allow axial movement of coupling mechanism 8 and actuating wire relative to jacket 22. The diameter of the lumen may also prevent radial displacement of one or both of boss 19 and stem 20. Accordingly, jacket 22 may hold together first portions 9, 10 of elongate member 4 and fix their axial positions, while allowing coupling mechanism 8 and the actuating wire to move relative to elongate member 4 and jacket 22. As discussed above, jacket 22 may also be configured to insulate coupling mechanism 8.

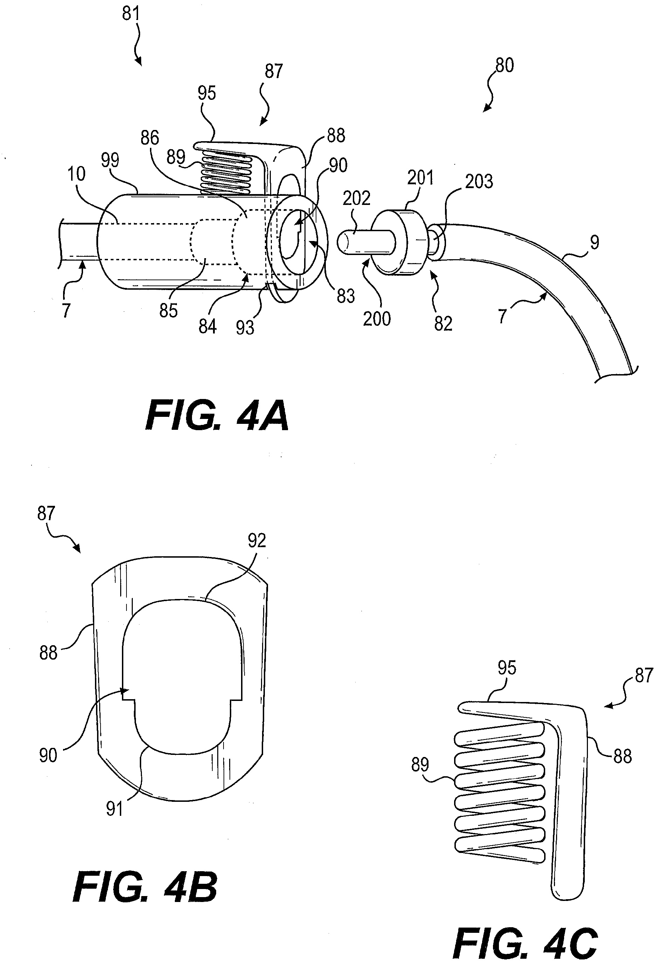

Fig. 4A illustrates a perspective view of another coupling mechanism 80, according to an exemplary disclosed embodiment. Coupling mechanism 80 may include a locking apparatus 81 coupled to second portion 10 of elongate member 4 and an insert apparatus 82 coupled to first portion 9 of elongate member 4. Locking apparatus 81 may be configured to engage and disengage with insert apparatus 82, and thus, operably couple and disconnect proximal handle 3 and distal tool 2.

Locking apparatus 81 may comprise a generally tubular structure 99 including an opening 83 and a cavity 84. Cavity 84 may include a first hollow section 85 and a second hollow section 86.

As shown in Fig. 4A, first hollow section 85 may include a diameter smaller than a diameter of second hollow section 86. Moreover, first hollow section 85 may be in communication with second portion 10 of elongate member 4. Second portion 10 may be connected to locking apparatus 81, with second portion 10 extending longitudinally through locking apparatus 81 and up to first hollow section 85. First hollow section 85 may be open to second portion 10. In certain embodiments, second portion 10 may travel through a bore in locking apparatus 81 that extends between a proximal end of locking apparatus 81 and first hollow section 85. Second portion 10 may also be fastened to the bore by any suitable mechanism, such as, for example, a friction fit, an interference fit, a weld, adhesives, fasteners, and the like. Second hollow section 86 may provide a passageway between first hollow section 85 and opening 83. A proximal end of second hollow section 86 may be open to first hollow section 85, and a distal end of second hollow section 86 may be open to opening 83. In other embodiments, first hollow section 85 and second hollow section 86 may have non-circular cross sections.

Although first hollow section 85 and second hollow section 86 are illustrated as having substantially continuous diameters, it should be appreciated that in certain other embodiments, one or both of the diameters of first hollow section 85 and second hollow section 86 may include a tapered configuration. For example, in some embodiments, the diameter of second hollow section 86 may be tapered towards a meeting point between first hollow section 85 and second hollow section 86.

Locking apparatus 81 may also include a locking component 87. Locking component 87 may be configured to secure first portion 9 of elongate member 4 when first portion 9 is disposed in cavity 84 of locking apparatus 81. As shown in Figs. 4A-4C, locking component 87 may include a holding plate 88 and a biasing mechanism 89.

Fig. 4B illustrates a front view of locking component 87, according to an exemplary disclosed embodiment. As shown in Fig. 4B, holding plate 88 may include a slot 90 having a first section 91 and a second section 92. Each of first section 91 and second section 92 may include a generally arcuate configuration, with first section 91 including a diameter smaller than a diameter of second section 92. As will be described in more detail below, second section 92 may include appropriate dimensions to allow passage of first portion 9 of elongate member 4 through cavity 84, while first section 91 may include appropriate dimensions to mate with and secure first portion 9. The size and shape of slot 90 in holding plate 88 is not limited to the embodiment shown in Fig. 4B.

Fig. 4C illustrates a side view of locking component 87, according to an exemplary disclosed embodiment. As shown in Figs. 4A and 4C, holding plate 88 may also include a lever 95. Lever 95 may be, for example, an outward protrusion of holding plate 88 substantially perpendicular to slot 90. Lever 95 may provide a surface for the operator to actuate locking component 87, and in some embodiments, may include an ergonomic configuration.

Biasing mechanism 89 may be disposed between lever 95 and an outer surface of tubular structure 99, and may be suitably fastened to one or both of lever 95 and the outer surface of tubular structure 99. Biasing mechanism 89 may be configured to provide an opposing force on locking component 87. For example, biasing mechanism 89 may provide an upward force on locking component 87. That is, the force may push up against lever 95. Biasing mechanism 89 may include any appropriate apparatus configured to provide a biasing force, such as, for example, a spring (e.g., a coil spring, leaf spring, cantilevered spring, or other biasing mechanism).

Locking component 87 may be actuatable relative to tubular structure 99. More specifically, and as shown in Fig. 4A, holding plate 88 may disposed in a channel 93 extending substantially perpendicular the longitudinal axis of tubular structure 99. Channel 93 may be appropriately positioned relative to tubular structure 99 such that holding plate 88 may extend through second hollow section 86. In addition, channel 93 may be appropriately dimensioned to allow movement of holding plate 88 relative to tubular structure 99. In addition, holding plate 88 may have variable thickness to help wedge it closed or open (as desired).

As alluded to above, biasing mechanism 89 may be positioned on the outer surface of tubular structure 99, with the force of biasing mechanism 89 biasing slot 90 of holding plate 88 to a predetermined position relative to second hollow section 86. Accordingly, upon a downward force on lever 95 (i.e., a force on lever 95 towards tubular structure 99) from, for example, the operator's thumb, holding plate 88 and slot 90 may move relative to channel 93, with a portion of holding plate 88 extending out of channel 93 in the direction of the force. When the force on lever 95 is released, the force from biasing member 89 may drive holding plate 88 upwards, and may consequently return slot 90 to the predetermined position relative to second hollow section 86.

Insert apparatus 82 may include an insertion rod 200 and a lug 201. Lug 201 may be disposed on an intermediate section of insertion rod 200 and may include a central bore into which rod 200 may be suitable fastened. In certain other embodiments, rod 200 may include two separate sections, each appropriately fastened to opposite sides of lug 201, or alternatively, rod 200 and lug 201 may be a continuous piece of a single material. Lug 201 may be merely a portion raised to enhance the connection with cavity 84. It is contemplated that, lug 201 may be bulbous, non-circular, may extend around only a portion of rod 200 and/or may be any suitable or desired shape and size. A proximal end 202 of rod 200 may include an appropriate size and shape to fit through cavity 84 and be positioned within first hollow section 85 of locking apparatus 81. While positioned within first hollow section 85, proximal end 202 of rod 200 may abut against second portion 10 of elongate member 4. Lug 201 may include an appropriate size and shape to fit through second hollow section 86 but may be restricted from entering first hollow section 85. For example, lug 201 may have a substantially cylindrical shape having a diameter larger than the diameter of first hollow section 85. A distal end 203 of rod 200 may include a size and shape to allow movement through second hollow section 86 and also to fit into first section 91 of slot 90 on holding plate 88. In addition, distal end 203 of rod 200 may be connected to first portion 9 of elongate member 4 by any suitable mechanism, such as, for example, a weld, adhesives, fasteners, and the like. In addition, one or more components of proximal end 202, e.g., rod 200 and/or lug 201, may have a polymer or elastomeric coating to improve the frictional engagement with cavity 84.

Insert apparatus 82 may comprise any suitable electrically-conductive material to transport electrical energy from second portion 10 of elongate member 4 to first portion 9 and distal tool 2. In certain embodiments, for example, one or both of rod 200 and lug 201 may comprise the electrically-conductive material. It should be appreciated, however, that in certain other embodiments first portion 9 may extend through and may be fastened to the central bore of lug 201, with a proximal end of first portion 9 configured to be inserted into first hollow section 85. First portion 9 may abut against second portion 10, and thus, electrical energy may be directly transferred therebetween.

To operably connect first and second portions 9, 10 of elongate member 4 using coupling mechanism 80, the operator may first actuate locking component 87 by depressing lever 95. Biasing member 89 may then collapse, and holding plate 88 may move relative to channel 93 such that slot 90 may be substantially centrally aligned relative to second hollow section 86. While lever 95 is depressed, insert apparatus 82 may be delivered though cavity 84 of tubular structure 99 and slot 90 of holding plate 88. Once proximal end 202 of rod 200 is positioned within first hollow section 85 and abuts second portion 10 of elongate member 4, the operator may release the force on lever 95. Holding plate 88 may return to its biased position, which may cause first portion 91 of slot 90 to abut against distal end 203 of rod 200.

Insert apparatus 82 may therefore be restricted from axial movement relative to cavity 84 by holding plate 88 and second portion 10 of elongate member 4. Holding plate 88 may prevent lug 201 from axially exiting cavity 84 in a distal direction, and second portion 10 of elongate member 4 may block axial translation of proximal end 202 of rod 200 in a proximal direction. Accordingly, as second portion 10 of elongate member 4 is proximally retracted by actuation of proximal handle 3, holding plate 88 may abut against a distal end of lug 201, and consequently, retract first portion 9 of elongate member 4 and distal tool 2 in the proximal direction. Moreover, as second portion 10 of elongate member 4 is distally advanced by actuation of proximal handle 3, second portion 10 may push against proximal end 202 of rod 200, and consequently, advance first portion 9 of elongate member 4 and distal tool 2 in the distal direction.

It should be appreciated that one or more elements of locking apparatus 81 may comprise a suitable electrically-insulating material incapable of conducting electricity. For example, tubular structure 99 and locking component 87 may comprise the insulating material. Accordingly, when the electrically-conductive components of insert apparatus 82 are positioned within cavity 84, tubular structure 99 and locking component 87 may electrically insulate insert apparatus 82 and second portion 10 to shield and prevent the discharge of stray electrical energy. Moreover, insulating material 7 may be coated over first portion 9 and the proximal end of second portion 10 that is external locking apparatus 81.

In certain other embodiments, coupling mechanism 80 may also be associated with an actuating wire (not shown) within elongate member 4, and may be configured to connect and disconnect portions of the actuating wire. The actuating wire may include a first section connected to distal tool 2, or a portion of distal tool 2, and extending through a lumen defined by first portion 9 and a second section connected to proximal handle 3 and extending through a lumen defined by second portion 10. Locking apparatus 81 may be connected to the second section of the actuating wire, and insert apparatus 82 may be connected to the first section of the actuating wire. Similar to the embodiments discussed above, insert apparatus 82 may be configured to engage and disengage with locking apparatus 81 to operably couple the actuating wire. For example, actuation of proximal handle 3 may move the actuating wire and distal tool 2 relative to elongate member 4, and an electrical connection may be formed between the electrical source (not shown), the actuating wire, coupling mechanism 80, and distal tool 2. Moreover, in such embodiments, jacket 22 may be slid over coupling mechanism 80, and may form a friction fit between first portion 9 and second portion 10 of elongate member 4. The lumen of jacket 22 may, however, include an appropriate diameter to allow axial movement of coupling mechanism 80 and actuating wire relative to jacket 22. Accordingly, jacket 22 may hold together first portions 9, 10 of elongate member 4 and fix their axial positions, while allowing coupling mechanism 80 and the actuating wire to move relative to elongate member 4 and jacket 22.

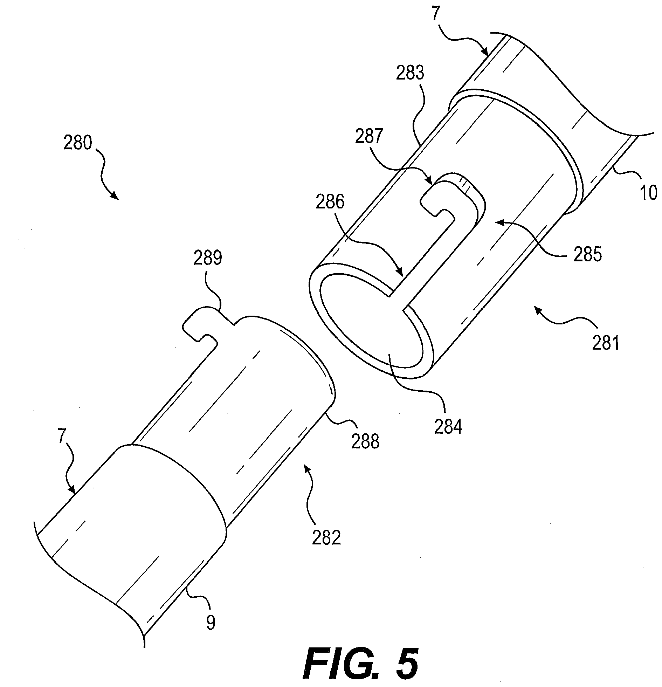

Fig. 5 illustrates a perspective view of another coupling mechanism 280, according to an exemplary disclosed embodiment. Coupling mechanism 280 may include a lock portion 281 coupled to second portion 10 of elongate member 4 and a key portion 282 coupled to first portion 9 of elongate member 4. Lock portion 281 may be configured to engage and disengage with key portion 282, and thus, operably couple and disconnect proximal handle 3 and distal tool 2.

Lock portion 281 may include a generally tubular-shaped member 283 having a lumen 284 and a key slot 285. Key slot 285 may be defined on an outer surface of tubular-shaped member 283 and may open into lumen 284. Key slot 285 may also include a delivery channel 286 open at a distal face of tubular-shaped member 283 and extending proximally to a key entrance 287.

Lock portion 281 may be connected to second portion 10 of elongate member 4 by any suitable mechanism, such as, for example, a weld, adhesives, fasteners, and the like. In addition, lock portion 281 may comprise any suitable electrically-conductive material to transport electrical energy from the electrical source (not shown) and second portion 10 to key portion 282. In certain embodiments, lock portion 281 and second portion 10 may be a single, continuous piece of the electrically-conductive material.

Key portion 282 may include a body 288 and a protrusion 289 extending radially from body 288. Similar to lock portion 281, key portion 282 may be connected to first portion 9 of elongate member 4 by any suitable mechanism, such as, for example, a weld, adhesives, fasteners, and the like. Furthermore, key portion 282 may comprise a suitable electrically-conductive material to transport electrical energy from lock portion 281 to first portion 9 and distal tool 2. It should also be appreciated that key portion 282 may instead be connected to second portion 10, and lock portion 281 may be connected to first portion 9. It should further be appreciated that protrusion 289, channel 286, and key entrance 287 are not limited to the shape shown. It is contemplated that key entrance 287 may be sized to fit tightly around protrusion 289 and the frictional engagement of such a tight fit may be enhanced with an elastomeric or polymer coating.

Body 288 may include an outer diameter smaller than a diameter of lumen 284. Moreover, protrusion 289 may include an appropriate thickness (measured relative to a circumference of body 288) such that protrusion 289 may fit into and move relative to delivery channel 286. Accordingly, when lock portion 281 and key portion 282 are positioned such that protrusion 289 is axially aligned with delivery channel 286, key portion 282 may be configured to be inserted into lumen 284 of lock portion 281.

Protrusion 289 and key entrance 287 may share a substantially similar shape, with the shape of protrusion 289 being smaller than the shape of key entrance 287. Protrusion 289 may be delivered through delivery channel 286 and to key entrance 287. Once protrusion 289 is substantially circumferentially aligned with key entrance 287, body 288 may be rotated such that protrusion 289 enters key entrance 287. In the embodiment of Fig. 5, for example, body 288 may be rotated counterclockwise relative to lock portion 281 (i.e., into the page) to have protrusion 289 enter lumen 284 of tubular-shaped member 283 via key entrance 287. Body 288 may be further rotated, and protrusion 289 may contact an inner wall defining lumen 284 to form a friction or interference fit therebetween. As such, key portion 282 may be secured to lock portion 281. To disconnect coupling mechanism 280, the friction or interference fit between protrusion 289 and the inner wall defining lumen 284 may be released by rotating body 288 in an opposite direction (e.g., clockwise relative to lock portion 281) such that protrusion 289 exits key entrance 287. Body 288 may be distally advanced such that protrusion 289 exits key slot 285 through delivery channel 286, resulting in key portion 282 and first portion 9 being decoupled from lock portion 281 and second portion 10.

As alluded to above, the secure connection between lock portion 281 and key portion 282 may operably connect proximal handle 3 and distal tool 2. That is, as second portion 10 of elongate member 4 is proximally retracted by actuation of proximal handle 3, the connection between lock portion 281 and key portion 282 may allow first portion 9 of elongate member 4 and distal tool 2 to be retracted in the proximal direction. Moreover, as second portion 10 of elongate member 4 is distally advanced by actuation of proximal handle 3, the connection between lock portion 281 and key portion 282 may allow first portion 9 of elongate member 4 and distal tool 2 to be advanced in the distal direction. The connection between lock portion 281 and key portion 282 also provides an electrical pathway between the electrical source (not shown) and distal tool 2.

Although not illustrated in Fig. 5, it should be appreciated that jacket 22 may be positioned over coupling mechanism 280 to insulate coupling mechanism 280, in a similar manner as discussed above in the embodiment of Figs. 3A-3C. Additionally, first and second portions 9, 10 may be coated with insulating material 7, and certain sections of lock portion 281 and key portion 282 may be selectively coated with an insulating material, similar to the embodiment of Figs. 3A-3C.

In some embodiments, lock portion 281 and key portion 282 may both comprise an insulating material. A distal end of second portion 10 may be disposed within lumen 284 of lock portion 281, and key portion 282 may define a lumen through which a proximal end of first portion 9 may extend. Accordingly, as key portion 282 is delivered into lumen 284 of lock portion 281, rotated, and secured, the distal end of second portion 10 and the proximal end of first portion 9 may abut against each other to form the electrical connection. The electrical connection between first portion 9 and second portion 10 may consequently be insulated by being surrounded by lock portion 281 and key portion 282.

In certain other embodiments, coupling mechanism 280 may be associated with an actuating wire (not shown) within elongate member 4, and may be configured to connect and disconnect portions of the actuating wire. The actuating wire may include a first section connected to distal tool 2, or a portion of distal tool 2, and extending through a lumen defined by first portion 9 and a second section connected to proximal handle 3 and extending through a lumen defined by second portion 10. Lock portion 281 may be connected to the second section of the actuating wire, and key portion 282 may be connected to the first section of the actuating wire. Similar to the embodiments discussed above, key portion 282 may be configured to engage and disengage with lock portion 281 to operably couple the actuating wire. For example, actuation of proximal handle 3 may move the actuating wire and distal tool 2 relative to elongate member 4, and an electrical connection may be formed between the electrical source (not shown), the actuating wire, coupling mechanism 280, and distal tool 2. Moreover, in such embodiments, jacket 22 may be slid over coupling mechanism 280, and may form a friction fit between first portion 9 and second portion 10 of elongate member 4. The lumen of jacket 22 may, however, include an appropriate diameter to allow axial movement of coupling mechanism 280 and actuating wire relative to jacket 22. Accordingly, jacket 22 may hold together first portions 9, 10 of elongate member 4 and fix their axial positions, while allowing coupling mechanism 280 and the actuating wire to move relative to elongate member 4 and jacket 22. As discussed above, jacket 22 may also be configured to insulate coupling mechanism 280.

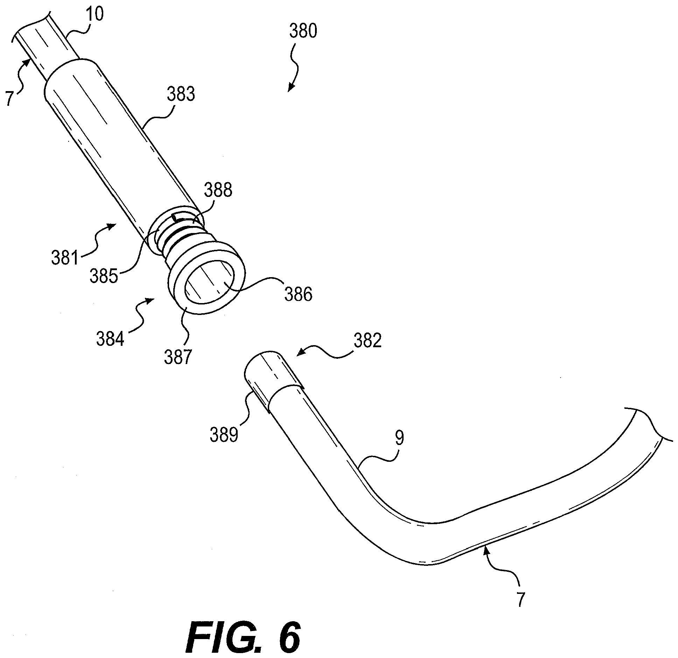

Fig. 6 illustrates a perspective view of yet another coupling mechanism 380, according to an exemplary disclosed embodiment. Coupling mechanism 380 may include a securing apparatus 381 connected to second portion 10 of elongate member 4 and a plug apparatus 382 connected to first portion 9 of elongate member 4. Plug apparatus 382 and securing apparatus 381 may be respectively connected to first and second portions 9, 10 by any suitable mechanism, such as, for example, a weld, adhesives, fasteners, and the like. Securing apparatus 381 may be configured to engage and disengage with plug apparatus 382, and thus, operably couple and disconnect proximal handle 3 and distal tool 2. It is contemplated that plug apparatus 382 may include any cross sectional shape.

Coupling mechanism 380 may comprise a suitable push-pin locking configuration. In such a configuration, for example, securing apparatus 381 may include a tubular body 383 defining a lumen and a release mechanism 384 coupled to tubular body 383. A plurality of clamping members (not shown) may be disposed within the lumen of tubular body 383. The clamping members may include any suitable structures configured to move between a first configuration constricting passage through the lumen of tubular body 383 and a second configuration permitting passage through the lumen of tubular body 383. In certain embodiments, the clamping members may include, as examples, ball bearings, arms, teeth, studs, and the like, configured to radially contract towards a central longitudinal axis of tubular body 383 and collapse away from the central longitudinal axis. The clamping members may be biased in the contracted configuration and may be moved to the collapsed configuration.

Release mechanism 384 may include a tubular member 385 disposed within the lumen of tubular body 383. Tubular member 385 may include a channel 386 extending therethrough and opening into the lumen of tubular body 383. Tubular member 385 may also include a collar portion 387 positioned external the lumen of tubular body 383 and having a diameter larger than a diameter of tubular body 383. Collar portion 387 may be appropriately sized to be restricted from entering the lumen of tubular body 383. Release mechanism 384 may also include a biasing mechanism 388, such as, for example, a spring, appropriately fastened to a distal face of tubular body 383 and a proximal end of collar portion 387. Biasing mechanism 388 may bias tubular member 385 to a predetermined position relative to the lumen of tubular body 383. For example, biasing mechanism 388 may position a proximal end of tubular member 385 distal to the clamping members within the lumen of tubular body 383.

Tubular member 385 may be proximally advanced relative to tubular body 383 by pressing on collar portion 387 and overcoming the biasing force of biasing mechanism 388. The operator may push collar portion 387 towards the distal face of tubular body 383, collapsing biasing mechanism 388, and thereby proximally moving tubular member 385 within the lumen of tubular body 383. In certain applications, collar portion 387 may restrict the amount of proximal movement of tubular member 385 by abutting against the distal face of tubular body 383. Tubular member 385 may then push away and/or collapse the clamping members to permit passage through the lumen of tubular body 383. By releasing the proximal force on collar portion 383, biasing mechanism 388 may return tubular member 385 to its predetermined position, and the clamping mechanism may radially contract to restrict passage through the lumen of tubular body 383.

Furthermore, one or more components of securing apparatus 381 may comprise any suitable electrically-conductive material to transport electrical energy from the electrical source (not shown) and second portion 10 to plug apparatus 382.

Plug apparatus 382 may include a linking body 389 appropriately connected to first portion 9 of elongate member 4. Linking body 389 and first portion 9 may include an appropriate size to fit through channel 386 of tubular member 385 and the lumen of tubular body 383. In addition, linking body 389 may be configured to engage with the clamping mechanism of securing apparatus 381. The clamping mechanism may be configured to constrict around and grasp linking body 389, thereby securing first portion 9 of elongate member 4 to securing apparatus 381. It should be appreciated that in some embodiments, linking body 389 may include suitable structural features to facilitate the engagement with the clamping mechanism, such as, for example, grooves, bumps, and the like. Furthermore, plug apparatus 382 may comprise a suitable electrically-conductive material to transport electrical energy from securing apparatus 381 to first portion 9 and distal tool 2.

To operably connect first and second portions 9, 10 of elongate member 4 using coupling mechanism 380, the operator may first actuate securing apparatus 381 by depressing collar portion 387. Biasing mechanism 388 may then collapse and tubular member 385 may proximally move relative to the lumen of tubular body 383 such that the clamping mechanism may be pushed away and/or collapsed to provide passage through the lumen of tubular body 383. While collar portion 387 is depressed, plug apparatus 382 may be delivered though channel 386 of tubular member 385 and into the lumen of tubular body 383. Linking body 389 may then be appropriately positioned within the lumen of tubular body 383 relative to the clamping mechanism. For example, suitable stops and/or restrictors may be disposed within the lumen of tubular body 383 to indicate that the clamping mechanism surrounds linking body 389. Once linking body 389 is appropriately positioned, the operator may release the force on collar portion 387. Tubular member 385 may be retracted to its predetermined position, and the clamping mechanism may constrict around and grasp linking body 389. Accordingly, securing apparatus 381 may be connected to plug apparatus 382, thus coupling first and second portions 9, 10 of elongate member 4 together.

Consequently, proximal handle 3 and distal tool 2 may be operably coupled. That is, as second portion 10 of elongate member 4 is proximally retracted by actuation of proximal handle 3, the connection between securing apparatus 381 and plug apparatus 382 may allow first portion 9 of elongate member 4 and distal tool 2 to be retracted in the proximal direction. Moreover, as second portion 10 of elongate member 4 is distally advanced by actuation of proximal handle 3, the connection between securing apparatus 381 and plug apparatus 382 may allow first portion 9 of elongate member 4 and distal tool 2 to be advanced in the distal direction. The connection between securing apparatus 381 and plug apparatus 382 may also provide an electrical pathway between the electrical source (not shown) and distal tool 2.

Although not illustrated in Fig. 6, it should be appreciated that jacket 22 may be positioned over coupling mechanism 380 to insulate coupling mechanism 380, in a similar manner as discussed above in the embodiment of Figs. 3A-3C. Additionally, first and second portions 9, 10 may be coated with insulating material 7, and certain sections of securing apparatus 381 and plug apparatus 382 may be selectively coated with an insulating material, similar to the embodiment of Figs. 3A-3C.

In some embodiments, securing apparatus 381 may comprise an insulating material, and first portion 9 may be directly connected to second portion 10. That is, a distal end of second portion 10 may be disposed within the lumen of tubular body 383, and a proximal end first portion 9 may be delivered into the lumen and secured to securing apparatus 381. The proximal end of first portion 9 and the distal end of second portion 10 may abut against each other to form the electrical connection. The electrical connection between first portion 9 and second portion 10 may consequently be insulated by being surrounded by securing apparatus 381. In addition, the sections of first and second portions 9, 10 external securing apparatus 381 may be coated with insulating material 7.

In certain other embodiments, coupling mechanism 380 may be associated with an actuating wire (not shown) within elongate member 4, and may be configured to connect and disconnect portions of the actuating wire. The actuating wire may include a first section connected to distal tool 2, or a portion of distal tool 2, and extending through a lumen defined by first portion 9 and a second section connected to proximal handle 3 and extending through a lumen defined by the second portion 10. Securing apparatus 381 may be connected to the second section of the actuating wire, and plug apparatus 382 may be connected to the first section of the actuating wire. Similar to the embodiments discussed above, plug apparatus 382 may be configured to engage and disengage with securing apparatus 381 to operably couple the actuating wire. For example, actuation of proximal handle 3 may move the actuating wire and distal tool 2 relative to elongate member 4, and an electrical connection may be formed between the electrical source (not shown), the actuating wire, coupling mechanism 380, and distal tool 2. Moreover, in such embodiments, jacket 22 may be slid over coupling mechanism 380, and may form a friction fit between first portion 9 and second portion 10 of elongate member 4. The lumen of jacket 22 may, however, include an appropriate diameter to allow axial movement of coupling mechanism 380 and actuating wire relative to jacket 22. Accordingly, jacket 22 may hold together first portions 9, 10 of elongate member 4 and fix their axial positions, while allowing coupling mechanism 380 and the actuating wire to move relative to elongate member 4 and jacket 22. As discussed above, jacket 22 may also be configured to insulate coupling mechanism 380.

Although the embodiments described above illustrate axial movement of elongate member 4 and distal tool 2, it should also be appreciated that the disclosed coupling mechanism may facilitate any other appropriate movement of elongate member 4 and distal tool 2, such as, for example, lateral deflection.

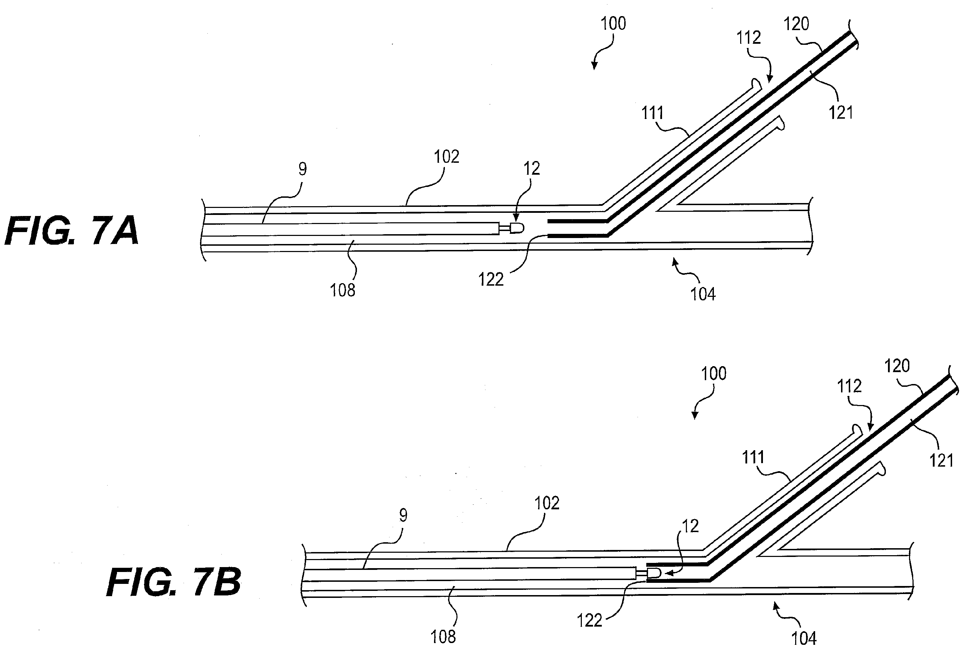

Figs. 7A and 7B schematically illustrate the delivery of elongate member 4 through endoscopic device 100, according to an exemplary disclosed embodiment. Figs. 7A and 7B particularly illustrate the proximal delivery of first portion 9 of elongate member 4 through elongate member 102. In such an embodiment, elongate member 102 may include a side port 111. Side port 111 may be disposed on proximal end 104 of elongate member 102 and may be angled with an opening 112 facing a proximal direction. Additionally, Figs. 7A and 7B illustrate elongate member 4 associated with coupling mechanism 8. That is, Figs. 7A and 7B show linkage apparatus 12 of coupling mechanism 8 connected to first portion 9 of elongate member 4. It should be appreciated, however, that any of coupling mechanisms 80, 280, and 380 may be applicable.

As shown in Fig. 7A, a guide tube 120 may be delivered through opening 112 of port 111 and into lumen 108 of elongate member 102. Guide tube 120 may comprise any suitable flexible material such that guide tube 120 may bend and substantially conform to the shape of lumen 108. Guide tube 120 may also comprise a channel 121 and a distal face 122 opening into channel 121. First portion 9 of elongate member 4 may be proximally delivered into lumen 108 via distal end 106 (Fig. 1) of elongate member 102, with linkage apparatus 12 facing guide tube 120.

As shown in Fig. 7B, first portion 9 of elongate member 4 may be proximally advanced through lumen 108 until linkage apparatus 12 is inserted through distal face 122 and into channel 121 of guide tube 120. The operator may hold the position of guide tube 120 as linkage apparatus 12 is inserted into channel 121. Additionally, or alternatively, the operator may distally advance guide tube 120 through lumen 108 to engage linkage apparatus 12. It should be appreciated that channel 121 may be appropriately dimensioned such that linkage apparatus 12 is held by guide tube 120 when inserted into channel 121 via, for example, a friction or interference fit. For instance, channel 121 may include a diameter smaller than a thickness of linkage apparatus 12 to effectuate the engagement between linkage apparatus 12 and guide tube 120.

Once linkage apparatus 12 is secured to guide tube 120, guide tube 120 and first portion 9, may be proximally retracted through side port 111 until linkage apparatus 12 exits opening 112 of side port 111. The operator may then disengage guide tube 120 and linkage apparatus 12, and may connect together first portion 9 and second portion 10 of elongate member 4 by engaging holding apparatus 11 to linkage apparatus 12 (Fig. 3). Accordingly, proximal handle 3 may be operably coupled to distal tool 2.

Medical device 1 may provide a number of features. For example, the ability to disassemble distal tool 2 from proximal handle 3 via coupling mechanism 8, 80, 280, 380 may allow medical device 1 to be used in a variety of different sized endoscopic devices. That is, for certain endoscopic devices having working channels too small to accommodate the delivery of distal tool 2 and/or proximal handle 3 therethrough, coupling mechanism 8, 80, 280, 380 may allow medical device 1 to still be used with such endoscopic devices. More specifically, first portion 9 and second portion 10 of elongate member 4 may first be disconnected from each other, thus decoupling distal tool 2 and proximal handle 3. First portion 9 or second portion 10 of elongate member 4 may then be delivered through the working channel, rather than the much larger distal tool 2 or proximal handle 3. First portion 9 and second portion 10 may be reconnected via coupling mechanism 8, 80, 280, 380, and thus, distal tool 2 and proximal handle 3 may be operably coupled, with distal tool 2 disposed on a distal end of endoscopic device, and proximal handle 3 disposed on a proximal end of endoscopic device. Accordingly, coupling mechanism 8, 80, 280, 380 may increase the applicability of medical device 1 with numerous endoscopic devices. In addition, coupling mechanism 8, 80, 280, 380 may provide the ability to readily customize distal tool 2 and/or proximal handle 3 of medical device 1. For instance, in customizing distal tool 2, coupling mechanism 8, 80, 280, 380 may allow, for example, a cap or a hood to be switched for a snare loop, a knife, a probe, jaws, or any other medical tool, by disconnecting first portion 9 from second portion 10 of elongate member 4, and connecting to second portion 10 another elongate member portion having a different medical tool coupled thereto. Moreover, proximal handle 3 may be switched with a different proximal handle having, for example, components to effectuate lateral deflection of elongate member 4 and/or different electrical conduction capabilities, by disconnecting second portion 10 from first portion 9 of elongate member 4, and connecting to first portion 9 another elongate member portion having a different proximal handle coupled thereto.

Any aspect set forth in any embodiment may be used with any other embodiment set forth herein. Every device and apparatus set forth herein may be used in any suitable medical procedure, may be advanced through any suitable body lumen and body cavity, and may be used to access tissue from any suitable body portion. For example, the apparatuses and methods described herein may be used through any natural body lumen or tract, including those accessed orally, vaginally, rectally, nasally, urethrally, or through incisions in any suitable tissue.

It will be apparent to those skilled in the art that various modifications and variations can be made in the disclosed systems and processes without departing from the scope of the invention. Other embodiments of the invention will be apparent to those skilled in the art from consideration of the specification and practice of the invention disclosed herein. It is intended that the specification and examples be considered as exemplary only. The following disclosure identifies some other exemplary embodiments.

In any of the embodiments described herein, when a locking sheath is utilized, an inner portion of the sheath may include an electrically conductive component or coating to assist in the conduction of electricity across the joint. It is contemplated that the devices described herein may be monopolor or bipolar. It is also contemplated that any of the connections described herein may be designed to allow fluid flow in or out based on, for example, hollow cores, grooves on surfaces, and/or other suitable mechanisms. It should further be appreciated that the elongate member not only conducts electricity but may also, in addition or alternatively, actuate an end-effector, steer or manipulate a distal end of the elongate member, or both. In such embodiments, it is contemplated that actuation may be affected via one or more actuation wires disposed relative to the elongate member as is known in the art.

## Claims

1. A medical device, comprising:
a proximal handle (3);
a distal tool (2);
an elongate member (4) connecting the distal tool to the proximal handle and including a first portion (9) and a second portion (10); and
a coupling mechanism (8) configured to selectively connect and disconnect the first and second portions of the elongate member, the coupling mechanism including a linkage apparatus (12) coupled to the first portion of the elongate member and a holding apparatus coupled to the second portion of the elongate member, **characterized in that** the linkage apparatus includes a boss (19) and a stem (20), the boss being larger than the stem, and the holding apparatus includes a lumen (16) configured to receive at least a portion of the linkage apparatus (12), a first slot (14), and a second slot (15), the first and second slots being open to the lumen (16), and the first slot being circumferentially offset from the second slot such that once the boss is inserted into the first slot, the stem is configured to be inserted into the lumen of the holding apparatus via the second slot.

2. The medical device of claim 1, wherein the elongate member is configured to conduct electrical energy to the distal tool.

3. The medical device of claim 1, wherein the first slot is larger than the second slot.

4. The medical device of claim 1, wherein the first slot is connected to the second slot.

5. The medical device of claim 4, wherein:
the boss has a radial dimension smaller than the first slot and larger than the second slot; and
the stem has a radial dimension smaller than the first and second slots.

6. The medical device of claim 1, wherein the boss is larger than the second slot.

## Patentansprüche

1. Medizinprodukt, das aufweist:
einen proximalen Griff (3);
ein distales Werkzeug (2);
ein längliches Teil (4), das das distale Werkzeug mit dem proximalen Griff verbindet und einen ersten Abschnitt (9) sowie einen zweiten Abschnitt (10) aufweist; und
einen Koppelmechanismus (8), der so konfiguriert ist, dass er den ersten und zweiten Abschnitt des länglichen Teils selektiv verbindet und trennt, wobei der Koppelmechanismus eine Anlenkungsvorrichtung (12), die mit dem ersten Abschnitt des länglichen Teils gekoppelt ist, und eine Haltevorrichtung aufweist, die mit dem zweiten Abschnitt des länglichen Teils gekoppelt ist, **dadurch gekennzeichnet, dass** die Anlenkungsvorrichtung eine Nabe (19) und einen Schaft (20) aufweist, wobei die Nabe größer als der Schaft ist, und die Haltevorrichtung ein Lumen (16), das so konfiguriert ist, dass es mindestens einen Abschnitt der Anlenkungsvorrichtung (12) aufnimmt, einen ersten Schlitz (14) und einen zweiten Schlitz (15) aufweist, wobei der erste und zweite Schlitz zum Lumen (16) offen sind und der erste Schlitz vom zweiten Schlitz über den Umfang so versetzt ist, dass bei eingesetzter Nabe in den ersten Schlitz der Schaft so konfiguriert ist, dass er in das Lumen der Haltevorrichtung über den zweiten Schlitz eingesetzt wird.

2. Medizinprodukt nach Anspruch 1, wobei das längliche Teil so konfiguriert ist, dass es elektrische Energie zum distalen Werkzeug leitet.

3. Medizinprodukt nach Anspruch 1, wobei der erste Schlitz größer als der zweite Schlitz ist.

4. Medizinprodukt nach Anspruch 1, wobei der erste Schlitz mit dem zweiten Schlitz verbunden ist.

5. Medizinprodukt nach Anspruch 4, wobei:
die Nabe eine Radialabmessung hat, die kleiner als der erste Schlitz und größer als der zweite Schlitz ist; und
der Schaft eine Radialabmessung hat, die kleiner als der erste und zweite Schlitz ist.

6. Medizinprodukt nach Anspruch 1, wobei die Nabe größer als der zweite Schlitz ist.

## Revendications

1. Dispositif médical comprenant :
une poignée proximale (3) ;
un outil distal (2) ;
un élément allongé (4) raccordant l'outil distal à la poignée proximale et comprenant une première partie (9) et une seconde partie (10) ; et
un mécanisme de couplage (8) configuré pour raccorder et déconnecter sélectivement les première et seconde parties de l'élément allongé, le mécanisme de couplage comprenant un appareil de liaison (12) couplé à la première partie de l'élément allongé et un appareil de support couplé à la seconde partie de l'élément allongé, **caractérisé en ce que** l'appareil de liaison comprend un bossage (19) et une tige (20), le bossage étant plus grand que la tige, et l'appareil de support comprend une lumière (16) configurée pour recevoir au moins une partie de l'appareil de liaison (12), une première fente (14) et une seconde fente (15), les première et seconde fentes étant ouvertes sur la lumière (16), et la première fente étant décalée de manière circonférentielle de la seconde fente de sorte qu'une fois que le bossage est inséré dans la première fente, la tige est configurée pour être insérée dans la lumière de l'appareil de support via la seconde fente.

2. Dispositif médical selon la revendication 1, dans lequel l'élément allongé est configuré pour amener l'énergie électrique à l'outil distal.

3. Dispositif médical selon la revendication 1, dans lequel la première fente est plus grande que la seconde fente.

4. Dispositif médical selon la revendication 1, dans lequel la première fente est raccordée à la seconde fente.

5. Dispositif médical selon la revendication 4, dans lequel :
le bossage a une dimension radiale inférieure à la première fente et supérieure à la seconde fente ; et
la tige a une dimension radiale inférieure aux première et seconde fentes.

6. Dispositif médical selon la revendication 1, dans lequel le bossage est plus grand que la seconde fente.
